(19) Europäisches Patentamt
European Patent Office
Office européen des brevets

(11) Numéro de publication : **0 206 899**
**B1**

# (12) FASCICULE DE BREVET EUROPÉEN

(45) Date de publication du fascicule du brevet : **30.11.88**

(51) Int. Cl.⁴ : **C 07 C 67/08**, C 07 C 69/653

(21) Numéro de dépôt : **86401249.7**

(22) Date de dépôt : **10.06.86**

(54) **Procédé de fabrication de (méth)acrylates d'alkyles fluorés.**

(30) Priorité : **18.06.85 FR 8509206**

(43) Date de publication de la demande :
**30.12.86 Bulletin 86/52**

(45) Mention de la délivrance du brevet :
**30.11.88 Bulletin 88/48**

(84) Etats contractants désignés :
**AT BE CH DE GB IT LI LU NL SE**

(56) Documents cités :
**US-A- 3 287 399**
**US-A- 3 384 627**

(73) Titulaire : **NORSOLOR S.A.**
**Tour Aurore Place des Reflets**
**F-92080 Paris la Defense Cédex 5 (FR)**

(72) Inventeur : **Hurtel, Patrice**
**2, Chemin des Brasseurs Résidence Foch**
**F-57500 Saint Avold (FR)**

(74) Mandataire : **Tilloy, Anne-Marie**
**NORSOLOR Service Propriété Industrielle Tour**
**Aurore Place des Reflets Cédex 5**
**F-92080 PARIS LA DEFENSE 2 (FR)**

**EP 0 206 899 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

**Description**

La présente invention a pour objet un nouveau procédé de fabrication de (méth)acrylates d'alkyles fluorés.

On entend par (méth)acrylates d'alkyles fluorés les composés de formule :

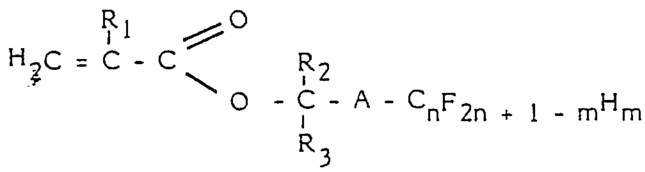

où

$R_1$ est un radical méthyle ou un atome d'hydrogène,

$R_2$ et $R_3$, identiques ou différents, sont choisis parmi H, $CH_3$, $CH_2$-$CH_3$ et $CF_3$,

A est choisi parmi $CF_2$ et CFH,

n est compris entre 1 et 19,

m est compris entre 0 et n.

Plusieurs voies de synthèse de ces composés sont actuellement bien connues.

La première est une réaction d'estérification entre un acide (méth)acrylique et un alcool fluoré. Elle est décrite notamment dans la demande de brevet japonaise 054511 du 30.03.83, pour la synthèse du méthacrylate de 2,2,2-trifluoroéthyle selon laquelle on fait réagir l'acide méthacrylique et le trifluoro-2,2,2 éthanol en présence d'un large excès d'acide sulfurique. Le temps de cette réaction est très long. Les auteurs de ce brevet annoncent 75 heures de réaction à 75 °C pour un rendement qui n'est que de 73 %.

Une deuxième voie de synthèse consiste à faire réagir un chlorure d'acide (méth)acrylique et un alcool fluoré. Les conditions opératoires de cette réaction sont complexes. En effet, il faut empêcher l'acide chlorhydrique formé lors de la réaction de s'additionner sur les doubles liaisons, notamment celle du (méth)acrylate. C'est pourquoi la réaction doit être effectuée en présence d'une substance organique réagissant avec l'acide chlorhydrique, par exemple la pyridine, pour former un sel que l'on doit ensuite extraire du milieu réactionnel. Un autre inconvénient de cette réaction réside dans la facilité avec laquelle les chlorures d'acide acrylique s'hydrolysent en présence d'humidité.

Le brevet américain 3.384.627 décrit la préparation d'acrylates d'alkyles fluorés par réaction, en présence d'hydroquinone, d'un alcool fluoré sur un mélange résultant de l'addition d'anhydride perfluoroacétique sur l'acide acrylique, ce qui donne lieu à la formation d'anhydride acrylique « in situ ». Cette méthode nécessite l'addition de l'anhydride perfluoroacétique ; les temps de réaction sont longs pour un rendement qui n'est que de 46-47 %.

La présente invention a pour objet un nouveau procédé de synthèse de (méth)acrylates d'alkyles fluorés ne comportant pas les inconvénients des procédés connus et permettant d'atteindre des rendements élevés.

Plus précisément, la présente invention concerne un nouveau procédé de fabrication de (méth)acrylates d'alkyles fluorés caractérisé en ce que l'on fait réagir un anhydride (méth)acrylique, de formule :

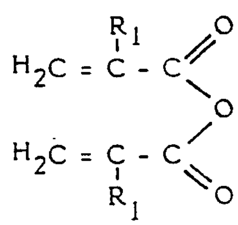

où $R_1$ est soit un radical méthyle, ou un atome d'hydrogène avec un dérivé fluoré d'alcool choisi parmi ceux de formule :

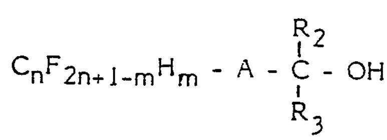

où :

$R_2$ et $R_3$, identiques ou différents, sont choisis parmi H, $CH_3$, $CH_2$-$CH_3$, $CF_3$

A est choisi parmi $CF_2$ et CFH,
n est compris entre 1 et 19,
m est compris entre 0 et n,
en présence d'au moins un inhibiteur de polymérisation et d'au moins un catalyseur acide, le rapport molaire de l'anhydride (méth)acrylique sur le dérivé fluoré d'alcool étant compris entre 1 et 1,2, puis on sépare le (méth)acrylate d'alkyles fluorés obtenu.

Selon un mode de réalisation préféré de l'invention, la séparation est effectuée par distillation. La distillation peut être conduite sous pression réduite et de manière fractionnée. Toutefois, si la température d'ébullition du (méth)acrylate d'alkyles fluorés et celle de l'acide (méth)acrylique formé dans le procédé selon l'invention sont proches, à savoir un écart de l'ordre de 10 °C ou inférieur, on effectue alors la séparation du méthacrylate d'alkyles fluorés de la manière suivante : on ajoute de l'eau dans le milieu réactionnel obtenu après réaction et on extrait l'acide (méth)acrylique dans la phase aqueuse. Eventuellement, on peut éliminer les traces d'acide (méth)acrylique restant par neutralisation à l'aide par exemple de soude puis on sépare par décantation la phase organique. On distille ensuite la phase organique afin d'obtenir une phase pure de (méth)acrylate d'alkyles fluorés.

De préférence, pendant toute la durée de la réaction selon l'invention, la température du milieu réactionnel est maintenue comprise entre 20 °C et la température d'ébullition du dérivé fluoré d'alcool. En raison de l'exothermicité de la réaction selon l'invention, on procède de préférence de la manière suivante :

On introduit dans un premier temps le dérivé fluoré d'alcool, un ou plusieurs inhibiteurs de polymérisation et un ou plusieurs catalyseurs acides, et ensuite, après agitation, on introduit progressivement l'anhydride (méth)acrylique.

Les anhydrides (méth)acryliques concernés par le procédé selon l'invention sont notamment l'anhydride acrylique, l'anhydride méthacrylique.

Parmi les alcools fluorés concernés par la présente invention, on peut citer notamment le trifluoro 2,2,2 éthanol, le tétrafluoro 2,2,3,3, propanol 1, l'hexafluoro 2,2,3,4,4,4, butanol 1.

Parmi les catalyseurs acide utilisables dans le procédé selon l'invention, on peut citer notamment l'acide sulfurique, l'acide paratoluène sulfonique, l'acide phosphorique, l'acide chlorhydrique, des résines cationiques ou des acides de Lewis comme par exemple le chlorure de Zinc, le chlorure de Fe (III). Ces catalyseurs acides sont introduits dans le milieu réactionnel dans un rapport molaire, par rapport à l'anhydride (méth)acrylique, supérieur ou égal à $10^{-4}$, et de préférence compris entre $10^{-3}$ et $10^{-2}$. Comme inhibiteur de polymérisation, on peut utiliser notamment la phénothiazine, le tertiobutylcatéchol, l'éther méthylique de l'hydroquinone, l'hydroquinone, le bleu de méthylène, le sulfate de cuivre, le sulfate de fer, dans un rapport molaire, par rapport à l'anhydride (méth)acrylique, supérieure ou égale à $10^{-4}$.

Le procédé selon l'invention est avantageux à plus d'un titre.

Le procédé selon l'invention est très simple, ce qui facilite son exploitation sur un site industriel.

Les temps de réaction sont de préférence compris entre 10 et 120 minutes.

Les rendements réalisés à l'aide du procédé selon l'invention sont élevés, et le plus souvent supérieurs à 90 %.

Les exemples donnés ci-dessous à titre indicatif permettront de mieux comprendre l'invention.

### Exemple I : Préparation du méthacrylate de 2,2,2 trifluoroéthyle

Dans un réacteur muni d'un système d'agitation mécanique et surmonté d'un réfrigérant, on introduit la charge suivante :

600 g de trifluoro 2,2,2 éthanol,
0,808 g de phénothiazine,
1,19 g d'acide sulfurique (100 %).

Après homogénéisation de cette charge à température ambiante, on introduit 1 016,4 g d'anhydride méthacrylique en continu et à pression atmosphérique.

Pendant toute la durée de la réaction, la température du milieu réactionnel est maintenue à 65 °C, environ.

Au bout d'une demi-heure, la réaction est terminée.

Le mélange réactionnel est alors distillé sous vide.

On distille en tête le trifluoro 2,2,2 éthanol qui passe entre 59 et 74 °C sous une pression de 0,33 bar, puis on récupère le méthacrylate de 2,2,2-trifluoroéthyle qui distille à 40 °C sous 0,066 bar. On recueille 984,44 g de méthacrylate de 2,2,2, trifluoroéthyle. Le rendement est de 97,6 %.

La distillation est terminée à 55 °C sous 0,02 bar afin d'éliminer l'acide méthacrylique formé lors de la réaction.

Le méthacrylate de 2,2,2 trifluoroéthyle recueilli possède les caractéristiques suivantes :

| | |
|---|---|
| densité à 25 °C : | 1,418 |
| tension de vapeur à 20 °C : | 0,026 bar |
| température d'ébullition sous 1,013 bar : | 100 °C |

Exemple II : Préparation du méthacrylate de 2,2,2 trifluoroéthyle

Le mode opératoire suivi dans cet exemple est en tout point identique à celui décrit dans l'exemple I, excepté l'acide sulfurique qui est introduit à raison de 0,6 g seulement.

Après distillation, on recueille 950,36 g de méthacylate de 2,2,2 trifluoroéthyle. Le rendement est de 94,3 %.

Exemple III : Préparation de méthacrylate de 2,2,2 trifluoroéthyle

Dans un réacteur muni d'un système d'agitation mécanique et surmonté d'un réfrigérant, on introduit la charge suivante :

1 500 g de trifluoro 2,2,2 éthanol,
1,92 g de bleu de méthylène,
2 405 g d'anhydride méthacrylique à 98,5 % en poids,
2,9 g d'acide sulfurique (100 %).

En suivant ensuite le mode opératoire défini dans l'exemple 1, on recueille après distillation 2 386,6 g de méthacrylate de 2,2,2 trifluoroéthyle méthacrylate. Le rendement est de 94,7 %.

Exemple IV : Préparation de l'acrylate de 2,2,2 trifluoroéthyle

Dans un réacteur muni d'un système d'agitation mécanique et surmonté d'un réfrigérant, on introduit la charge suivante :

200 g de trifluoro 2,2,2 éthanol,
0,47 g de sulfate de cuivre,
0,38 g d'acide sulfurique (100 %)
315 g d'anhydride acrylique.

Pendant toute la durée de la réaction entre le 2,2,2 trifluoroéthanol et l'anhydride acrylique, la température du milieu réactionnel est maintenue inférieure à 65 °C.

Au bout de 10 minutes, la réaction est terminée.

Le mélange réactionnel est alors distillé sous vide.

On distille d'abord le trifluoro 2,2,2 éthanol qui passe à 40 °C sous 0,33 bar puis on récupère l'acrylate de 2,2,2 trifluoroéthyle qui distille à 63 °C sous 0,33 bar. On recueille ainsi 295 g d'acrylate de 2,2,2 trifluoroéthyle. Le rendement est de 95,7 %.

Exemple V : Préparation de méthacrylate de 2,2,3,4,4,4 hexafluorobutyle

Dans un réacteur muni d'un système d'agitation mécanique et surmonté d'un réfrigérant, on introduit la charge suivante :

1 820 g d'hexafluoro-2,2,3,4,4,4 butanol 1,
1 694 g d'anhydride méthacrylique,
3,00 g d'acide sulfurique (100 %),
2,35 g de sulfate de fer.

On maintient la température à 65 °C pendant la réaction. Au bout d'une demi-heure, la réaction est terminée. On procède ensuite à la séparation du méthacrylate de 2,2,3,4,4,4-hexafluorobutyle formé.

On ajoute alors dans le milieu réactionnel 3 500 ml d'eau distillée et après agitation, on sépare par décantation la phase aqueuse de la phase organique.

Après distillation de la phase organique sous 0,066 bar et à 78 °C on obtient 2 420 g de méthacrylate de 2,2,3,4,4,4 hexafluorobutyle possédant les caractéristiques suivantes :

| | |
|---|---|
| densité à 25 °C : | 1,338 |
| indice de réfraction à 25 °C : | 1,3592 |

Le rendement de la réaction est de 96,8 %.

Exemple VI : Préparation d'acrylate de 2,2,3,4,4,4 hexafluorobutyle

Dans un réacteur muni d'un système d'agitation mécanique et surmontéd'un réfrigérant, on introduit la charge suivante :

910 g d'hexafluoro-2,2,3,4,4,4-butanol 1,
1,4 g d'acide sulfurique (100 %),
1,2 g de sulfate de cuivre,
puis 661,5 g d'anhydride acrylique.

On maintient la température à 65 °C pendant la réaction. Au bout d'un quart d'heure, la réaction est terminée.

La séparation de l'acrylate de 2,2,3,4,4,4 hexafluorobutyle est effectuée comme indiqué dans l'exemple V.

Après distillation sous 0,066 bar à 70 °C, on recueille 1 147 g d'acrylate de 2,2,3,4,4,4 hexafluorobutyle possédant les caractéristiques suivantes :

| | |
|---|---|
| densité à 25 °C : | 1 389 |
| indice de réfraction à 25 °C : | 1,3492 |

Le rendement de la réaction est de 97,2 %.

Exemple VII : Préparation de méthacrylate de 2,2,3,3 tétrafluoropropyle

Dans un réacteur muni d'un système d'agitation mécanique et surmonté d'un réfrigérant, on introduit la charge suivante :

1 340 g de tétrafluoro 2,2,3,3 propanol 1,
2,9 g d'acide sulfurique (100 %),
1,6 g de phénothiazine,
puis 1 617 g d'anhydride méthacrylique.

On maintient la température à 65 °C pendant la réaction. Au bout d'une demi-heure, la réaction est terminée. On procède alors à la séparation du méthacrylate de 2,2,3,3 tétrafluoropropyle formé. On ajoute dans le milieu réactionnel 2 000 ml d'eau distillée et après agitation, on sépare par décantation la phase aqueuse de la phase organique.

Après distillation de la phase organique sous 0,066 bar et à 73 °C, on recueille 1 959,5 g de méthacrylate de 2,2,3,3 tétrafluoropropyle possédant les caractéristiques suivantes :

| | |
|---|---|
| densité à 25 °C : | 1,255 |
| indice de réfraction à 25 °C : | 1,3723 |

Le rendement de la réaction est de 97 %.

Exemple VIII : Préparation de l'acrylate de 2,2,3,3 tétrafluoropropyle

Dans un réacteur muni d'un système d'agitation mécanique et surmonté d'un réfrigérant, on introduit la charge suivante :

1 340 g de tétrafluoro 2,2,3,3 propanol 1,
2,8 g d'acide sulfurique (100 %),
1,6 g de phénotiazine,
puis 1 384 g d'anhydride acrylique.

La température est maintenue à 65 °C pendant la réaction. Au bout d'une demi-heure, la réaction est terminée. On procède alors à la séparation de l'acrylate de 2,2,3,3 tétrafluoropropyle comme indiqué dans l'exemple VII.

Après distillation sous 0,066 bar à 63 °C, on recueille 1 844 g d'acrylate de 2,2,3,3 tétrafluoropropyle possédant les caractéristiques suivantes :

| | |
|---|---|
| densité à 25 °C : | 1,308 |
| indice de réfraction à 25 °C : | 1,3628 |

Le rendement de la réaction est de 98,1 %.

**Revendications**

1. Procédé de préparation de (méth)acrylates d'alkyles fluorés caractérisé en ce que l'on fait réagir un anhydride (méth)acrylique, de formule :

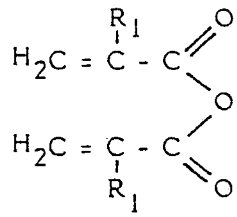

où $R_1$ est soit un radical méthyle, ou un atome d'hydrogène, avec un dérivé fluoré d'alcool choisi parmi ceux de formule :

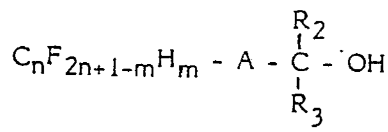

où

$R_2$ et $R_3$, identiques ou différents, sont choisis parmi H, $CH_3$, $CH_2$-$CH_3$, $CF_3$,

A est choisi parmi $CF_2$ et CFH,

n est compris entre 1 et 19,

m est compris entre 0 et n,

en présence d'au moins un inhibiteur de polymérisation et d'au moins un catalyseur acide, le rapport molaire de l'anhydride (méth)acrylique sur le dérivé fluoré d'alcool étant compris entre 1 et 1,2, puis on sépare le (méth)acrylate d'alkyles fluorés obtenu.

2. Procédé selon la revendication 1, caractérisé en ce que pendant toute la durée de la réaction entre l'anhydride (méth)acrylique et le dérivé fluoré d'alcool, la température du milieu réactionnel est maintenue comprise entre 20 °C et la température d'ébullition du dérivé fluoré d'alcool.

3. Procédé selon la revendication 1 ou 2, caractérisé en ce que l'on introduit tout d'abord le dérivé fluoré d'alcool, au moins un inhibiteur de polymérisation et au moins un catalyseur acide, et ensuite on introduit progressivement l'anhydride (méth)acrylique.

4. Procédé selon l'une quelconque des revendications 1 à 3, caractérisé en ce que la quantité de catalyseur acide introduite est telle que le rapport molaire de ce catalyseur sur l'anhydride (méth)acrylique est supérieure ou égale à $10^{-4}$.

5. Procédé selon l'une quelconque des revendications 1 à 4, caractérisé en ce que la quantité d'inhibiteur de polymérisation introduite est telle que le rapport molaire de cet inhibiteur sur l'anhydride (méth)acrylique est supérieure ou égale à $10^{-4}$.

6. Procédé selon l'une quelconque des revendications 1 à 5, caractérisé en ce que le (méth)acrylate d'alkyle fluoré obtenu est séparé par distillation.

**Claims**

1. Process for the preparation of fluoroalkyl (meth)acrylates, characterized in that a (meth) acrylic anhydride, of formula :

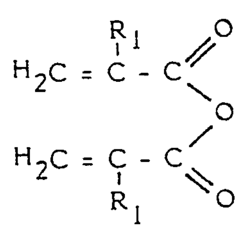

where $R_1$ is either a methyl radical or a hydrogen atom, is reacted with an alcohol fluoro derivative which is chosen from those of the formula :

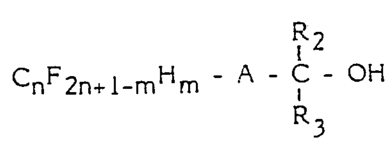

where

$R_2$ and $R_3$, which are identical or different, are chosen from H, $CH_3$, $CH_2$-$CH_3$ and $CF_3$,

A is chosen from $CF_2$ and CFH,

n is between 1 and 19, and

m is between 0 and n,

in the presence of at least one polymerization inhibitor and at least one acid catalyst, the molar ratio of the (meth)acrylic anhydride to the alcohol fluoro derivative being 1 and 1.2, and the fluoroalkyl (meth) acrylates obtained are then separated off.

2. Process according ta Claim 1, characterized in that, during the entire period of reaction between the (meth)acrylic anhydride and the alcohol fluoro derivative the temperature of the reaction mixture is maintained between 20 °C and the boiling point of the alcohol fluoro derivative.

3. Process according to Claim 1 or 2, characterized in that the alcohol fluoro derivative, at least one polymerization inhibitor and at least one acid catalyst are introduced first of all, and then the (meth) acrylic anhydride is introduced gradually.

4. Process according to any one of Claims 1 to 3, characterized in that the quantity of acid catalyst introduced is such that the molar ratio of this catalyst to the (meth)acrylic anhydride is greater than or equal to $10^{-4}$.

5. Process according to any one of Claims 1 to 4, characterized in that the quantity of polymerization inhibitor introduced is such that the molar ratio of this inhibitor to (meth)acrylic anhydride is greater than or equal to $10^{-4}$.

6. Process according to any one of Claims 1 to 5, characterized in that the fluoroalkyl (meth)acrylate obtained is separated off by distillation.

**Patentansprüche**

1. Verfahren zur Herstellung von fluorierten Alkyl (meth)acrylaten, dadurch gekennzeichnet, daß man ein (Meth) Acrylsäureanhydrid der Formel :

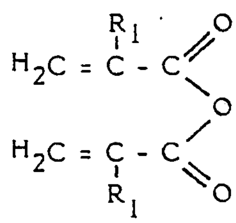

in der $R^1$ entweder ein Methylrest oder ein Wasserstoffatom ist, mit einer fluorierten Alkoholverbindung, die ausgewählt ist aus jenen der Formel :

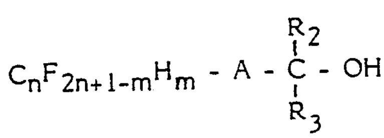

in der

$R_2$ und $R_3$, die identisch oder verschieden sind, ausgewählt sind aus der Gruppe bestehend aus H, $CH_3$, $CH_2$-$CH_3$ und $CF_3$,

A ausgewählt ist aus der Gruppe bestehend aus $CF_2$ und CFH,

n zwischen 1 und 19 beträgt und

m zwischen 0 und n beträgt

in Gegenwart mindestens eines Polymerisationsinhibitors und mindestens eines Säurekatalysators reagieren läßt, wobei das Molverhältnis des (Meth)Acrylsäureanhydrids zur fluorierten Alkoholverbindung zwischen 1 und 1,2 beträgt, und daß man anschließend die erhaltenen fluorierten Alkyl (meth)acrylate abtrennt.

2. Verfahren nach Anspruch 1, dadurch gekennzeichnet, daß während der gesamten Dauer der Reaktion zwischen dem (Meth)Acrylsäureanhydrid und der fluorierten Alkoholverbindung die Temperatur des Reaktionsmilieus zwischen 20 °C und der Siedetemperatur der fluorierten Alkoholverbindung gehalten wird.

3. Verfahren nach Anspruch 1 oder 2, dadurch gekennzeichnet, daß man zuallererst die fluorierte Alkoholverbindung, mindestens einen Polymerisationsinhibitor und mindestens einen Säurekatalysator und danach allmählich das (Meth)Acrylsäureanhydrid einbringt.

4. Verfahren nach irgendeinem der Ansprüche 1 bis 3, dadurch gekennzeichnet, daß die eingesetzte

Menge an Säurekatalysator derart ist, daß das Molverhältnis dieses Katalysators zum (Meth)Acrylsäureanhydrid größer als oder gleich $10^{-4}$ ist.

5. Verfahren nach irgendeinem der Ansprüche 1 bis 4, dadurch gekennzeichnet, daß die eingesetzte Menge an Polymerisationsinhibitor derart ist, daß das Molverhältnis dieses Inhibitors zum (Meth)Acrylsäureanhydrid größer als oder gleich $10^{-4}$ ist.

6. Verfahren nach irgendeinem der Ansprüche 1 bis 5, dadurch gekennzeichnet, daß das erhaltene fluorierte Alkyl (meth)acrylat durch Destillation abgetrennt wird.